# EUROPEAN PATENT APPLICATION

(11) **EP 1 977 706 A1**
(43) Date of publication of application: **08.10.2008**
(21) Application number: 08006733.3
(22) Date of filing: 02.04.2008
(51) Int. Cl.: A61B 18/12, A61B 18/14

(54) **Controller for flexible tissue ablation procedures**

(30) Priority: 03.04.2007 US 732271
(71) Applicant: Tyco Healthcare Group, LP, North Haven CT 06473 (US)
(72) Inventor: McPherson, James W., Boulder, CO 80301 (US); Puterbaugh, Lewis, Longmont, CO 80501 (US)
(74) Representative: HOFFMANN EITLE

(57) **Abstract**

An electrosurgical system including a generator configured to supply radiofrequency (RF) energy is disclosed. The system includes at least two electrodes configured to apply RF energy to tissue and at least one return electrode for returning the RF energy to the generator. The generator may operate in a first operational mode and at least one other operational mode. The system includes a controller configured to control the application of RF energy to each of the two or more electrodes and the return of RF energy to the generator. The controller includes a plurality of switching components configured to selectively switch the flow of the RF energy between the two or more electrodes and between the return electrode and at least one of the two or more electrodes based on the operational mode of the generator.

## Description

### BACKGROUND

### Technical Field

The present disclosure relates to electrosurgical apparatuses, systems and methods. More particularly, the present disclosure is directed to an electrosurgical system employing a controller configured for monopolar and/or bipolar ablation procedures utilizing an arbitrary number and/or combination of electrodes.

### Background of Related Art

Energy-based tissue treatment is well known in the art. Various types of energy (e.g., electrical, ultrasonic, microwave, cryo, heat, laser, etc.) are applied to tissue to achieve a desired result. Electrosurgery involves application of high radio frequency electrical current to a surgical site to cut, ablate, coagulate or seal tissue. In monopolar electrosurgery, a source or active electrode delivers radio frequency energy from the electrosurgical generator to the tissue and a return electrode carries the current back to the generator. In monopolar electrosurgery, the source electrode is typically part of the surgical instrument held by the surgeon and applied to the tissue to be treated. A patient return electrode is placed remotely from the active electrode to carry the current back to the generator.

The use of radiofrequency electrodes for ablation of tissue in a patient's body is known. In a typical situation, a radiofrequency electrode comprising an elongated, cylindrical shaft with a portion of its external surface insulated is inserted into the patient's body. The electrode typically has an exposed conductive tip, which is used to contact body tissue in the region where the heat lesion or ablation is desired. The electrode is connected to a radiofrequency power source, which provides radiofrequency voltage to the electrode, which transmits the radiofrequency current into the tissue near its exposed conductive tip. This current usually returns to the power source through a reference electrode, e.g., a return electrode, which may comprise a large area conductive contact connected to an external portion of the patient's body.

Conventional electrode systems are limited by the practical size of the lesion volumes they produce, typically due to the use of a single large electrode. An advantage of a multiplicity of smaller electrodes versus insertion of a single large electrode is that the smaller electrodes will produce less chance of hemorrhage. The arrangement of their geometry may also be tailored to the clinical application. Further, a configuration of radiofrequency electrodes that allows for the tailoring of the shape and size of the lesion obtained is desirable.

Additionally, electrosurgical generators used to energize conventional electrode systems require multiple RF amplifiers, each adapted for different operational modes (e.g., monopolar, bipolar, etc.). Each RF amplifier energizes an electrode based on the procedure for which it is configured to be used. This limitation makes electrosurgical generators costly, heavy, and overly complex.

### SUMMARY

The present disclosure relates to a radiofrequency (RF) electrosurgical system configured for monopolar and/or bipolar procedures by utilizing an arbitrary number of active and return electrodes for producing large ablation volumes in tissue or producing multiple ablation volumes during a single procedure. A method for using the electrosurgical system is also provided. The electrosurgical system includes an RF source, such as an electrosurgical generator, and a controller to direct RF energy delivery from a single generator output to a plurality of electrodes. By employing multiple electrodes in a single procedure, the electrosurgical system can create large lesions or can ablate two or more separate lesions simultaneously. The electrosurgical system of the present disclosure allows for the use of an arbitrary number of electrodes which allows the electrosurgical system to ablate volumes of various shapes and sizes.

The present disclosure also relates to a system for heat ablation of tissue in a patient and includes an RF source for supplying RF energy, at least two electrodes configured to apply RF energy to tissue, at least one return electrode for returning the RF energy to the RF source, and a controller configured to control the flow of RF energy between an arbitrary number of electrodes which may be energized either sequentially, in parallel, or in a bipolar activation between multiple electrodes. The controller utilizes switching components to divert RF current to any one or more selected electrodes depending on the method of procedure being performed (e.g., bipolar, monopolar, footswitch operation, etc.).

### BRIEF DESCRIPTION OF THE DRAWINGS

Various embodiments of the present disclosure are described herein with reference to the drawings wherein:
Fig. 1A is a schematic block diagram of a monopolar electrosurgical system in accordance with an embodiment of the present disclosure;
Fig. 1B is a schematic block diagram of a bipolar electrosurgical system in accordance with an embodiment of the present disclosure;
Fig. 2 is a schematic block diagram of a generator in accordance with an embodiment of the present disclosure;
Fig. 3A is a schematic illustration of a unit controller of an electrosurgical system configured for monopolar operation in accordance with an embodiment of the present disclosure;
Fig. 3B is a schematic illustration of a unit controller of an electrosurgical system configured for bipolar operation in accordance with an embodiment of the present disclosure;
Fig. 4A is a schematic illustration of a scaled up embodiment of the unit controller of Fig. 3A configured for monopolar operation in accordance with the present disclosure; and
Fig. 4B is a schematic illustration of a scaled up embodiment of the unit controller of Fig. 3B configured for bipolar operation in accordance with the present disclosure.

### DETAILED DESCRIPTION

Particular embodiments of the present disclosure are described hereinbelow with reference to the accompanying drawings. In the following description, well-known functions or constructions are not described in detail to avoid obscuring the present disclosure in unnecessary detail.

The present disclosure provides for a controller adapted to selectively divert treatment energy through and between an arbitrary number of electrodes in an electrosurgical system. The use of a multiplicity of N electrodes increases the overall conductive exposed tip area by which to send RF current for heating into the tissue. This increases the heating power that may be delivered and thus increases the size of the ablation volume possible. Further, the cooling capacity of a multiplicity of N electrodes also increases as the number N increases. Increasing the number of electrodes increases the cooling surface area near the electrodes. Thus, the heat sinking effect from a plurality of electrodes is greater than the heat sinking effect from a single electrode element. This enables the lesion size to be expanded accordingly.

An advantage of a multiplicity of smaller electrodes versus insertion of a single large electrode is that the smaller electrodes will produce less chance of hemorrhage. The arrangement of their geometry may also be tailored to the clinical application. Insertion of several small gauge electrodes is less painful, uncomfortable, and risk-inducing than insertion of one large, equivalent radiofrequency electrode. For example, insertion of a cluster of several 18 gauge or 1.25 mm diameter pointed radiofrequency electrodes into the liver produces very low risk of hemorrhage and low discomfort. Insertion of an equivalent, but much larger single electrode, which may have a diameter of, for example, 0.25" or 6.4 mm, would have a higher risk of hemorrhage and would be very uncomfortable for the patient if the electrode were inserted percutaneously.

A generator according to the present disclosure can perform monopolar and bipolar electrosurgical procedures, including vessel sealing procedures. The generator may include a plurality of outputs for interfacing with various electrosurgical instruments (e.g., a monopolar active electrode, return electrode, bipolar electrosurgical forceps, footswitch, etc.). Further, the generator includes electronic circuitry configured for generating radio frequency power specifically suited for various electrosurgical modes (e.g., cutting, blending, division, etc.) and procedures (e.g., monopolar, bipolar, vessel sealing, tissue ablation).

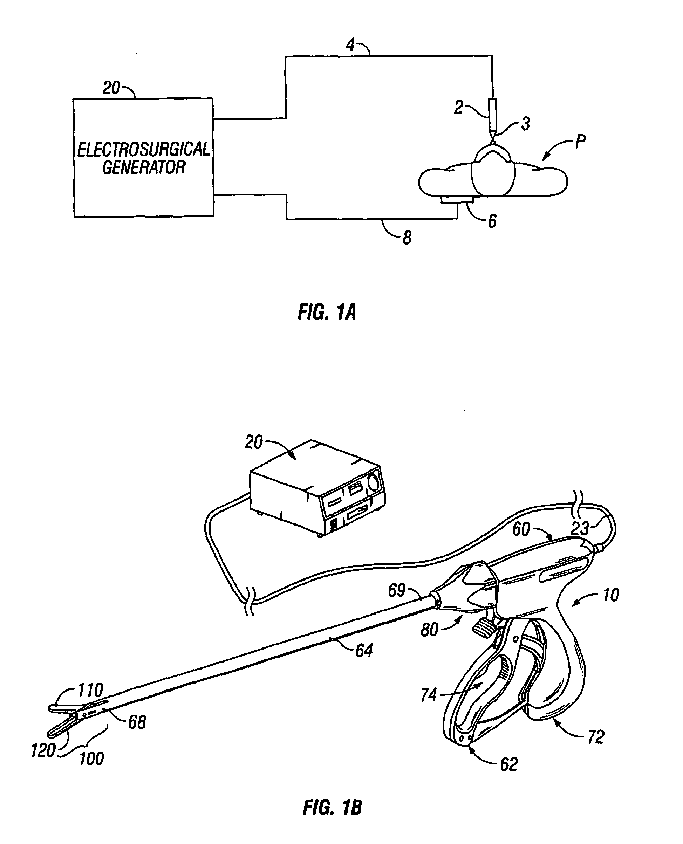

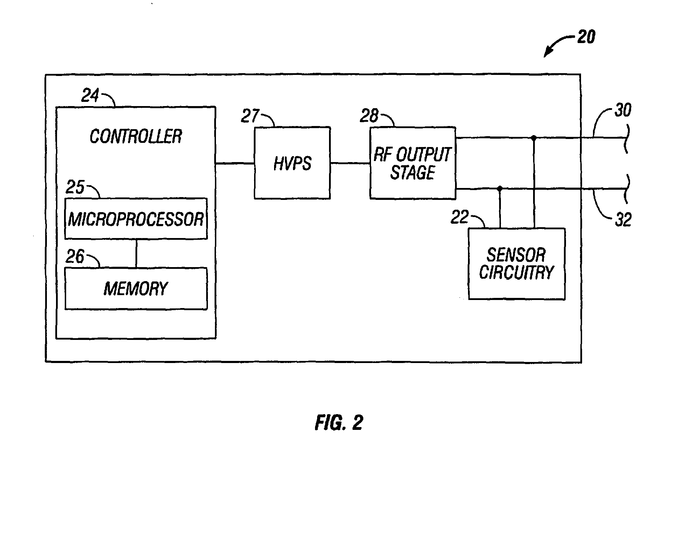

Fig. 1A is a schematic illustration of a monopolar electrosurgical system according to embodiments of the present disclosure. The system includes a monopolar electrosurgical instrument 2 including one or more active electrodes 3, which can be electrosurgical cutting probes, ablation electrode(s), etc. Electrosurgical RF energy is supplied to the instrument 2 by a generator 20 via a supply line 4, which is connected to an active terminal 30 (Fig. 2) of the generator 20, allowing the instrument 2 to coagulate, ablate and/or otherwise treat tissue. The energy is returned to the generator 20 through a return electrode 6 via a return line 8 at a return terminal 32 (Fig. 2) of the generator 20. The active terminal 30 and the return terminal 32 are connectors configured to interface with plugs (not explicitly shown) of the instrument 2 and the return electrode 6, which are disposed at the ends of the supply line 4 and the return line 8 respectively.

The system may include a plurality of return electrodes 6 that are arranged to minimize the chances of tissue damage by maximizing the overall contact area with the patient P. In addition, the generator 20 and the return electrode 6 may be configured for monitoring so-called "tissue-to-patient" contact to insure that sufficient contact exists therebetween to further minimize chances of tissue damage.

The present disclosure may be adapted for use with either monopolar or bipolar electrosurgical systems. Fig. 1B shows a bipolar electrosurgical system according to the present disclosure which includes an electrosurgical forceps 10 having opposing jaw members 110 and 120. The forceps 10 includes one or more shaft members having an end effector assembly 100 disposed at the distal end. The end effector assembly 100 includes two jaw members movable from a first position wherein the jaw members are spaced relative to on another to a closed position wherein the jaw members 110 and 120 cooperate to grasp tissue therebetween. Each of the jaw members includes an electrically conductive sealing plate (not shown) connected to the generator 20 through cable 23, which communicates electrosurgical energy through the tissue held therebetween. Cable 23 includes the supply and return lines coupled to the active and return terminals 30, 32, respectively (Fig. 2).

Those skilled in the art will understand that embodiments of the present disclosure may be adapted for use with either an endoscopic instrument or an open instrument. More particularly, forceps 10 generally includes a housing 60, a handle assembly 62, which mutually cooperate with the end effector assembly 100 to grasp and treat tissue. The forceps 10 also includes a shaft 64 which has a distal end 68 that mechanically engages the end effector assembly 100 and a proximal end 69 which mechanically engages the housing 60 proximate the rotating assembly 80. Handle assembly 62 includes a fixed handle 72 and a movable handle 74. Handle 74 moves relative to the fixed handle 72 to actuate the end effector assembly 100 and enable a user to grasp and manipulate tissue. More particularly, the jaw members 110 and 120 move in response to movement of the handle 74 from an open position to a closed position. Further details relating to one envisioned endoscopic forceps is disclosed in commonly-owned U.S. Patent No. 7,090,673 entitled "Vessel Sealer and Divider."

With reference to Figs. 1A and 1B, the generator 20 includes suitable input controls (e.g., buttons, activators, switches, touch screen, etc.) for controlling the generator 20. In addition, the generator 20 may include one or more display screens for providing the user with variety of output information (e.g., intensity settings, treatment complete indicators, etc.). The controls allow the user to adjust power of the RF energy, waveform, and other parameters to achieve the desired waveform suitable for a particular task (e.g., coagulating, tissue sealing, intensity setting, etc.). The instrument 2 or the forceps 10 may also include a plurality of input controls that may be redundant with certain input controls of the generator 20. Placing the input controls at the instrument 2 the forceps 10 allows for easier and faster modification of RF energy parameters during the surgical procedure without requiring interaction with the generator 20.

Fig. 2 shows a schematic block diagram of the generator 20 having a control component 24, a high voltage DC power supply 27 ("HVPS") and an RF output stage 28. The HVPS 27 is connected to a conventional AC source (e.g., electrical wall outlet) and provides high voltage DC power to an RF output stage 28, which then converts high voltage DC power into RF energy and delivers the RF energy to the active terminal 30. The energy is returned thereto via the return terminal 32.

In particular, the RF output stage 28 generates sinusoidal waveforms of high RF energy. The RF output stage 28 is configured to generate a plurality of waveforms having various duty cycles, peak voltages, crest factors, and other suitable parameters. Certain types of waveforms are suitable for specific electrosurgical modes. For instance, the RF output stage 28 generates a 100% duty cycle sinusoidal waveform in cut mode, which is best suited for ablating, fusing and dissecting tissue and a 1-25% duty cycle waveform in coagulation mode, which is best used for cauterizing tissue to stop bleeding.

The generator 20 may include a plurality of connectors to accommodate various types of electrosurgical instruments (e.g., instrument 2, electrosurgical forceps 10, etc.). Further, the generator 20 may be configured to operate in a variety of modes such as ablation, monopolar and bipolar cutting coagulation, etc. It is envisioned that the generator 20 may include a switching mechanism (e.g., relays) to switch the supply of RF energy between the connectors, such that, for instance, when the instrument 2 is connected to the generator 20, only the monopolar plug receives RF energy.

The control component 24 includes a microprocessor 25 operably connected to a memory 26, which may be volatile type memory (e.g., RAM) and/or non-volatile type memory (e.g., flash media, disk media, etc.). The microprocessor 25 includes an output port that is operably connected to the HVPS 27 and/or RF output stage 28 allowing the microprocessor 25 to control the output of the generator 20 according to either open and/or closed control loop schemes. Those skilled in the art will appreciate that the microprocessor 25 may be substituted by any logic processor (e.g., control circuit) adapted to perform the calculations discussed herein.

A closed loop control scheme is a feedback control loop wherein sensor circuit 22, which may include a plurality of sensors measuring a variety of tissue and energy properties (e.g., tissue impedance, tissue temperature, output current and/or voltage, etc.), provides feedback to the control component 24. Such sensors are within the purview of those skilled in the art. The control component 24 then signals the HVPS 27 and/or RF output stage 28, which then adjust DC and/or RF power supply, respectively. The control component 24 also receives input signals from the input controls of the generator 20 or the instrument 2. The control component 24 utilizes the input signals to adjust power outputted by the generator 20 and/or performs other control functions thereon.

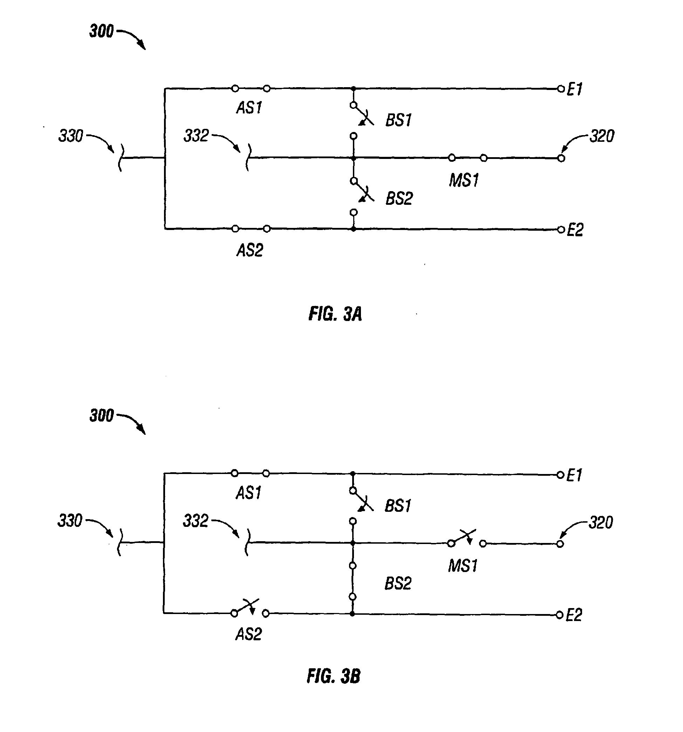

Figs. 3A - 3B show a controller 300 according to embodiments of the present disclosure configured for monopolar and bipolar modes of operation respectively. Controller 300 is adapted to divert RF energy through and between a pair of electrodes E1 and E2, which may be inserted into an organ of the human body. The electrodes E1 and E2 are individually coupled to the generator 20 (Figs. 1A-1B) by wires or cables (not shown). A reference electrode 320, e.g., a return electrode, is also shown, which may be placed in contact with the skin of a patient or the external surface of an organ. In embodiments, this serves as a path for return current from the generator 20 (Figs. 1A-1B) through electrodes E1 and E2.

According to one embodiment of the present disclosure, electrodes E1 and E2 may be placed in a single target, e.g., a tumor. The heating effect of the multiple electrodes is similar to that accomplished by one large single electrode. The individual electrodes E1 and E2 cause less trauma and are less likely to induce hemorrhaging when they penetrate an organ because of their smaller size. Yet, when they are connected to a radiofrequency voltage source, they represent an effectively much larger electrode. In this way, larger heat volumes, and therefore ablation sizes, may be achieved.

Controller 300 is connected to the active output of the generator 20 (Figs. 1A-1B) via an active terminal 330 and to the return output of the generator 20 via a return terminal 332. A pair of active switching components AS1 and AS2 are also shown, which are configured to control the flow of RF current between electrodes E1 and/or E2. The controller according to embodiments of the present disclosure need not be connected to the generator via an active terminal and a return terminal but, rather, may be integrated within the generator itself.

A pair of bipolar switching components BS1 and BS2 are also shown, which are configured to control the flow of RF current returning from at least one of the active electrodes E1 and/or E2 to the generator 20 (Fig. 1B) via the return terminal 332. A monopolar switching component MS1 allows for configuring the controller 300 to accommodate monopolar procedures, wherein monopolar switching component MS1 is closed, and bipolar procedures, wherein monopolar switching component MS1 is open. Figs. 3A and 3B show controller 300 configured for typical monopolar and bipolar procedures, respectively.

Fig. 3A shows controller 300 adapted for operation in a monopolar procedure according to embodiments of the present disclosure. During a typical monopolar procedure, for example, monopolar switching component MS1 and active switching components AS1 and AS2 may be closed while both bipolar switching components BS1 and BS2 are open. In this way, monopolar switching component MS1 may serve as a path for RF current returning from return electrode 320 to the generator 20 (Fig. 1A). RF current may be channeled to electrodes E1 and E2 sequentially or in parallel simultaneously allowing either or both of electrodes E1 and E2 to serve as active electrodes. As to be appreciated, electrode 320 may be embodied as a return electrode pad including, for example, a capacitive layer (not shown) on an outer conductive surface thereof or disposed between one or more conductive material layers (not shown), such as metallic foil which adhere to the patient and are configured to conduct electrosurgical RF energy back to the generator 20. Embodiments of electrode 320 as a return electrode pad may further include an adhesive material layer on a patient-contacting surface thereof. The adhesive material may be, but is not limited to, a polyhesive adhesive, a Z-axis adhesive, a water-insoluble, hydrophilic, pressure-sensitive adhesive, or any combinations thereof, such as POLYHESIVE™ adhesive manufactured by Valleylab, a division of Tyco Healthcare Co., of Boulder, Colorado. The adhesive may be conductive or dielectric. The adhesive material layer ensures an optimal surface contact area between the return electrode pad and the patient, thereby limiting the possibility of a patient burn.

Fig. 3B shows controller 300 adapted for operation in a bipolar procedure according to embodiments of the present disclosure. During a typical bipolar procedure, for example, electrode E1 may operate as the active electrode and electrode E2 may operate as the return electrode. In the present example, bipolar switching component BS1, active switching component AS2, and monopolar switching component MS1 are open. Conversely, active switching component AS1 and bipolar switching component BS2 are closed. In this way, active switching component AS1 serves as a path for RF current channeled to active electrode E1 from the generator 20 (Fig. 1B). Similarly, bipolar switching component BS2 serves as a path for RF current returning from electrode E2 to the generator 20 (Fig. 1B) via terminal 332 while monopolar switching component MS1 remains open thereby rendering electrode 320 inactive and/or unused.

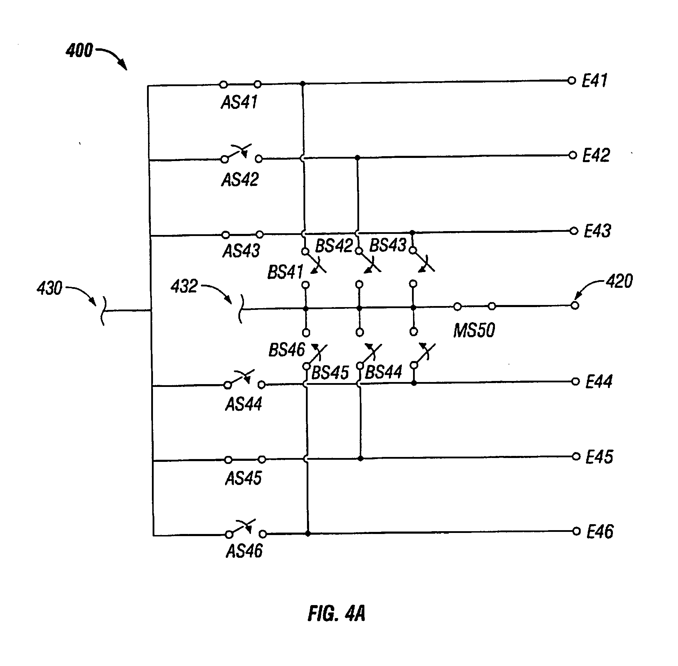

Controller 300 is merely illustrative of a "unit" controller in accordance with the present disclosure and may be scaled up to accommodate an arbitrary number of electrodes, as shown in Figs. 4A - 4B. For example, if N is the number of electrodes employed in a given procedure then embodiments of the present disclosure will require 2N + 1 switching components to construct the controller. Switching components according to embodiments of the present disclosure may be embodied as any suitable type of switch adopted to allow interruption of the flow of RF current through controller 300. Examples of such devices include, but are not limited to, toggles, relays, solid state switches, etc.

Figs. 4A - 4B show a controller 400 illustrating a scaled up embodiment of the unit controller 300 of Figs. 3A - 3B utilizing an arbitrary number of active and return electrodes. As previously discussed, employing N electrodes for use with the present disclosure will require 2N + 1 switching components. In the present examples illustrated in Figs. 4A - 4B, controller 400 utilizes six electrodes and, thus, requires thirteen switching components including six active switching components AS41 - AS46, six bipolar switching components BS41 - BS46, and a monopolar switching component MS50. A return electrode (or return pad) 420 is included for use in monopolar procedures to return RF current to the generator (Fig. 1A) via the return terminal, referenced in the present example as 432. Accordingly, monopolar switching component MS50 is closed during monopolar procedures. Electrodes E41 - E46 are supplied RF current from the generator 20 (Figs. 1A - 1B) via the active terminal, referenced in the present example as 430.

Fig. 4A shows controller 400 adapted for operation in a monopolar procedure according to embodiments of the present disclosure. During a typical monopolar procedure, for example, controller 400 may utilize any possible number and/or combination of active electrodes E41 - E46 wherein RF current is supplied to any and/or each of electrodes E41- E46 simultaneously or, alternatively, sequentially by the generator 20 (Fig. 1A). In the present example illustrated in Fig. 4A, electrodes E41, E43, and E45 may be utilized in parallel simultaneously or may be sequentially switched, leaving electrodes E42, E44, and E46 inactive and/or unused. In this configuration, active switching components AS41, AS43, and AS45 are closed to allow RF current to be channeled from the generator 20 (Fig. 1A) to active electrodes E41, E43, and E45 respectively. Active switching components AS42, AS44, and AS46 remain open, thereby preventing RF current from flowing to the remaining unused electrodes E42, E44, and E46. Monopolar switching component MS50 is closed to serve as a path for RF current returning from return electrode 420 to the generator 20 (Fig. 1A) via terminal 432. Accordingly, bipolar switching components BS47 - BS52 remain open during monopolar operation of controller 400 to allow for a direct path from return electrode (or return pad) 420 to return terminal 432. Although the present example illustrates a procedure utilizing a specific set of electrodes (E41, E43, and E45), it should be understood that the present example is illustrative only and an arbitrary number of electrodes and/or combination of electrodes may be employed as is warranted or desired by the user.

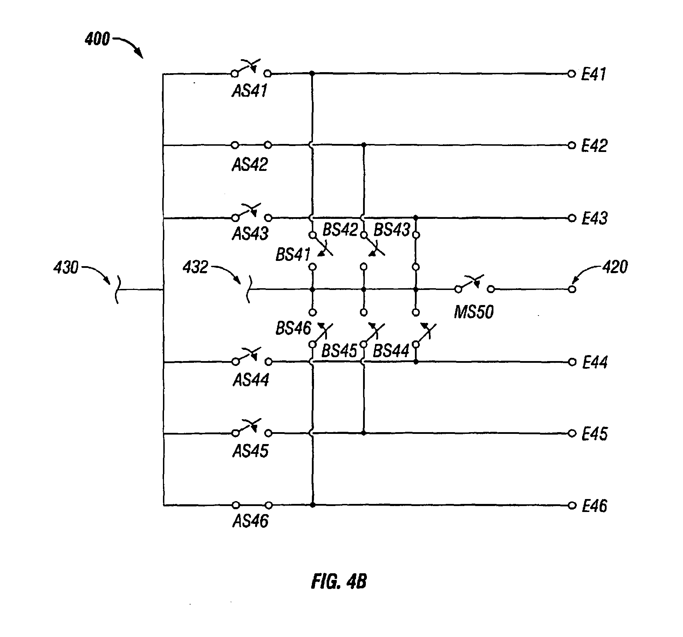

Fig. 4B shows controller 400 adapted for operation in a bipolar procedure according to embodiments of the present disclosure. During a typical bipolar procedure, for example, any one or more electrodes E41 - E46 may operate as either an active electrode configured to channel RF current to the patient (not shown), or alternatively as a return electrode (or return pad) configured to return RF current to the generator 20 (Fig. 1B). As such, return electrode (or return pad) 420 is rendered unnecessary and, thus, inactive and/or unused during bipolar procedures. Accordingly, monopolar switching component MS50 is open during the procedure of the present example to prevent the flow of RF current through monopolar switching component MS50. It should be appreciated with respect to procedures illustrated in the present example, that when any one of active switching components AS41 - AS46 are closed, the corresponding electrode E41 - E46 is chosen to serve as an active electrode, configured to channel RF current therethrough. Likewise, when any one of bipolar switching components BS41 - BS46 are closed, the corresponding electrode E41 - E46 is chosen to serve as a return electrode. In the present example, electrodes E42 and E46 are being utilized simultaneously as active electrodes and electrode E43 is being used as the return electrode, leaving electrodes E41, E44, and E45 unused. Accordingly, active switching components AS42 and AS46 are closed to serve as a path for the flow of RF current from the generator 20 (Fig. 1B) to electrodes E42 and E46, respectively. Active switching components AS41, AS43, AS44, and AS45 are open, thereby preventing the flow of RF current to return electrode E43 and the remaining unused electrodes E41, E44, and E45. Further, bipolar switching component BS43 is closed to serve as a path for the flow of RF current returning from electrode E43 to the generator 20 (Fig. 1B).

Although the present example illustrates a procedure utilizing a specific set of active electrodes (E42 and E46) and a specific return electrode (E43), it should be appreciated that the present example is illustrative only and an arbitrary number of electrodes and/or combination of active and return electrodes may be employed as is warranted or desired by the user. For example, any one or more of electrodes E41 - E46 may be employed as an active electrode. Likewise, any one or more of electrodes E41 - E46 may alternatively be employed as a return electrode.

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

## Claims

1. An electrosurgical system comprising:
an electrosurgical generator configured to supply radiofrequency (RF) energy, the generator being further configured to operate in a first operational mode and at least one other operational mode;
at least two electrodes configured to apply RF energy to tissue;
at least one return electrode configured to return the RF energy to the electrosurgical generator; and
a controller configured to selectively control the flow of the RF energy returning to the electrosurgical generator and the application of the RF energy to each of the at least two electrodes, the controller comprising a plurality of switching components configured to selectively switch the flow of the RF energy between each of the at least two electrodes and between the return electrode and at least one of the at least two electrodes based on the operational mode of the electrosurgical generator.

2. The system according to claim 1, wherein at least one of the at least two electrodes is configured to return the RF energy to the electrosurgical generator.

3. The system according to any preceding claim, wherein the controller is configured to apply RF energy sequentially to each of the at least two electrodes during the first operational mode of the generator.

4. The system according to any preceding claim, wherein the controller is configured to simultaneously apply RF energy in parallel to each of the at least two electrodes during the first operational mode of the generator.

5. The system according to any preceding claim, wherein the controller is configured to apply RF energy to each of the at least two electrodes individually during a second operational mode of the generator.

6. The system according to any preceding claim, wherein the number of switching components is based on the equation (2 * N) + 1, wherein N represents the number of electrodes utilized in the system.

7. The system according to any preceding claim, wherein the first operational mode of the generator is configured for at least one of a monopolar electrosurgery and a bipolar electrosurgery.

8. The system according to any preceding claim, wherein the electrosurgical generator is configured to supply radiofrequency (RF) energy in a first operational mode configured for monopolar heat ablation and a second operational mode configured for bipolar heat ablation.

9. The system according to claim 9, wherein an arbitrary number of electrodes are utilized.
